# EUROPEAN PATENT SPECIFICATION

(11) **EP 4 051 115 B1**
(45) Date of publication and mention of the grant of the patent: **11.11.2026**
(21) Application number: 20803973.5
(22) Date of filing: 23.10.2020
(51) Int. Cl.: A61B 5/395

(54) **MUSCLE RELAXATION MONITORING APPARATUS AND CALIBRATION PROCESSING METHOD**
VORRICHTUNG ZUR ÜBERWACHUNG DER MUSKELENTSPANNUNG UND KALIBRIERUNGSVERARBEITUNGSVERFAHREN
APPAREIL DE SURVEILLANCE DE RELÂCHEMENT MUSCULAIRE ET PROCÉDÉ DE TRAITEMENT D'ÉTALONNAGE

(30) Priority: 31.10.2019 JP 2019198590
(43) Date of publication of application: 07.09.2022
(73) Proprietor: Nihon Kohden Corporation, Shinjuku-ku Tokyo 161-8560 (JP); Takagi, Shunichi, Tokyo 1610033 (JP)
(72) Inventor: YOSHIHARA Hiroshi, Tokorozawa-shi, Saitama 359-0037 (JP); KITAMURA Shigeyoshi, Tokorozawa-shi, Saitama 359-0037 (JP); NAGASE Kazuya, Tokorozawa-shi, Saitama 359-0037 (JP); IWATA Shunji, Tokorozawa-shi, Saitama 359-0037 (JP); TAKAGI Shunichi, Tokyo 1610033 (JP)
(74) Representative: Grünecker Patent- und Rechtsanwälte PartG mbB
(86) International application number: PCT/JP2020/039941
(87) International publication number: WO 2021/085333

(56) References cited:
- US-A1- 2014 107 524
- US-A1- 2019 008 453
- US-A1- 2019 223 764

## Description

### Technical Field

The presently disclosed subject matter relates to a muscle relaxation monitoring apparatus for identifying the muscle relaxation state or awake state of a subject such as a patient, and also to a calibration processing method.

### Background

PTL 1 described below discloses a technique for monitoring the muscle relaxation state of the subject due to a muscle relaxant agent that is administered during surgery. In the anesthesia monitoring system disclosed in PTL 1 described below, a predetermined stimulation mode such as the Train-Of-Four (TOF) method is set, and the muscle relaxation state of the subject is quantitatively monitored based on a muscle contraction force of the observation portion that is induced when an electrical stimulation of a predetermined current value is applied to a peripheral nerve leading to a portion (muscle) to be observed that is a part of the body of the subject.

### Citation List

### Patent Literature

[PTL 1] JP-T-2015-506245
US 2019/0223764 Al provides quantitative neuromuscular blockade sensing systems and methods. A neuromuscular lead assembly for a neuromuscular blockade monitoring is described and may include a base supporting a plurality of electrodes.

### Summary

### Technical Problem

When the system of PTL 1 is to be used, in order to correctly know the muscle relaxation state of the subject, the electrical stimulation to be applied to the subject must be set to the supramaximal stimulation (a stimulation exceeding the maximal stimulation that causes all muscle fibers in the observation portion, to contract, and increased by, for example, 10% to 20% from the maximal stimulation) by which, even when the current value is further increased, the muscle response is not increased. In the system, therefore, a calibration process is performed before a muscle relaxant agent is administered, and a stimulation current value that causes the supramaximal stimulation according to the muscle relaxation state of the subject is detected.

In a calibration process, a stimulation current value that causes the supramaximal stimulation is searched by applying a stimulation while decreasing the current value in steps of a predetermined value (for example, 2 mA) in every predetermined period (for example, 1 Hz) from the stimulation current value of the maximal stimulation (for example, 60 mA) that is the maximum value of the stimulation current in the apparatus configuration. However, the supramaximal stimulation of a subject is frequently in a range of 30 mA to 60 mA. In the case of a subject in whom the stimulation current value of the supramaximal stimulation is 30 mA, for example, there is a problem in that a calibration process requires a long time period.

In the prior art, a calibration process is started at the maximal stimulation current value. In the case where the subject is an infant or a neonatal infant, particularly, there is therefore a problem in that a physical damage due to an electrical stimulation is severe as compared with the case of an adult, and an excessive burden is imposed.

As described above, the prior art has room for improvement in the points that the supramaximal stimulation of a subject is detected in a short time period, and that an excessive burden is imposed on a subject when the supramaximal stimulation is to be detected.

The presently disclosed subject matter has been conducted in view of the above-described problems of the prior art. It is an object of the presently disclosed subject matter to provide a muscle relaxation monitoring apparatus and calibration processing method that, while reducing the burden imposed on a subject, can detect a stimulation current value that causes the supramaximal stimulation according to the subject, in a short time period.

### Solution to Problem

This invention provides a muscle relaxation monitoring apparatus according to claim 1 and a calibration processing method according to claim 12.

The muscle relaxation monitoring apparatus of the presently disclosed subject matter including a calibration processing section for acquiring a stimulation current value of a supramaximal stimulation exceeding a maximal stimulation of a muscle of a subject, wherein the calibration processing section sets an initial stimulation current value that is set to be smaller than a maximum value of a stimulation current, as a starting stimulation current value, determines, as a current value variable process, one of: a first current value variable process that increases by a value corresponding to a predetermined step current value at a predetermined stimulation timing; and a second current value variable process that decreases by the value corresponding to the step current value at the stimulation timing, detects, as a first peak value, an amplitude peak value of an electric signal that is based on a stimulation response of the muscle due to an electrical stimulation of a first stimulation current value that is a stimulation current value obtained before the stimulation current value is varied by the value corresponding to the step current value in the determined current value variable process, detects, as a second peak value, an amplitude peak value of an electric signal that is based on a stimulation response of the muscle due to an electrical stimulation of a second stimulation current value that is varied by the value corresponding to the step current value from the first stimulation current value, based on the determined current value variable process, at a stimulation timing that is next to the stimulation timing of the first stimulation current value, detects a stimulation current value of a maximal stimulation of the subject, based on a result of a comparison of the first peak value and the second peak value, and acquires a stimulation current value that is obtained by adding the step current value to the stimulation current value of the maximal stimulation of the subject, as the stimulation current value of the supramaximal stimulation of the subject.

### Effects of the Invention

According to the presently disclosed subject matter, it is possible to, while reducing the burden imposed on a subject, detect a stimulation current value that causes the supramaximal stimulation according to the subject, in a short time period.

### Brief Description of Drawings

[fig.1]FIG. 1 illustrates the configuration of a muscle relaxation monitoring apparatus of an embodiment of the presently disclosed subject matter.
[fig.2]FIG. 2 conceptually illustrates a calibration process that is executed in a calibration processing section.
[fig.3]FIG. 3 is a flowchart of a series of process operations of the muscle relaxation monitoring apparatus.
[fig.4]FIG. 4 is a flowchart of process operations in a calibration process.
[fig.5]FIG. 5 is a flowchart of an electric current value increasing/comparing process in the calibration process.
[fig.6]FIG. 6 is a flowchart of an electric current value decreasing/comparing process in the calibration process.

### Description of Embodiments

Hereinafter, an embodiment of the presently disclosed subject matter will be described in detail with reference to the drawings. The presently disclosed subject matter is not limited by the following embodiment. All of other modes, embodiments, operation techniques, and the like that can be conceived by those skilled in the art or the like based on the embodiment should be included in the scope of the presently disclosed subject matter.

### <Configuration of apparatus>

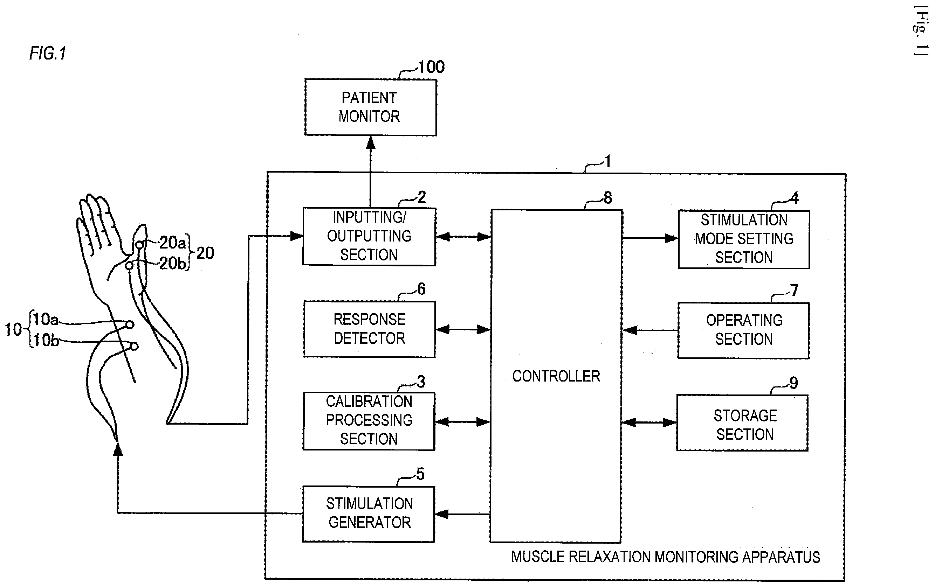

Referring to FIG. 1, first, the configuration of a muscle relaxation monitoring apparatus 1 of the embodiment of the presently disclosed subject matter will be described.

The muscle relaxation monitoring apparatus 1 is wiredly or wirelessly communicably connected to a patient monitor 100 (such as a bedside monitor, a transport monitor, or a medical monitor). Display contents corresponding to a stimulation response value that is detected by the muscle relaxation monitoring apparatus 1 are displayed on the patient monitor 100.

A stimulation electrode section 10 and a detection electrode section 20 are connected to the muscle relaxation monitoring apparatus 1. The muscle relaxation monitoring apparatus 1 applies an electrical stimulation of a predetermined current value, to a nerve in an observation portion of the subject through the stimulation electrode section 10. The muscle relaxation monitoring apparatus 1 acquires an electric signal that is based on a stimulation response (muscle response) of the observation portion due to the electrical stimulation, through the detection electrode section 20.

The muscle relaxation monitoring apparatus 1 of the embodiment is a so-called electromyography (EMG) that quantitatively detects the muscle relaxation state of the subject based on the action potential of muscle fibers in the observation portion that is induced when an electrical stimulation of the predetermined current value is applied to a peripheral nerve leading to a muscle which is the observation portion of the subject.

The stimulation electrode section 10 is configured by a pair of stimulation electrodes 10a, 10b that are attachable to and detachable from the skin surface above the peripheral nerve leading to the muscle which is the observation portion of the subject, and performs an electrical stimulation of a preset predetermined current value on the peripheral nerve leading to the muscle in the observation portion. In the stimulation electrode section 10, in the case where the polarity of the stimulation electrode 10a is plus, for example, that of the stimulation electrode 10b is minus. In the stimulation electrode section 10, in the case where the observation portion is the adductor pollicis muscle, for example, both the stimulation electrodes 10a, 10b are attached to the skin surface above the ulnar nerve leading to the adductor pollicis muscle, while being separated by a predetermined interval from each other. The stimulation electrode section 10 is connectable to and disconnectable from the muscle relaxation monitoring apparatus 1, and, when a failure or the like occurs, can be replaced with another one.

The detection electrode section 20 is configured by a pair of detection electrodes 20a, 20b that are attachable to and detachable from the skin surface of the observation portion of the subject, detects the action potential of the muscle in the observation portion due to the electrical stimulation performed by the stimulation electrode section 10, as a stimulation response, and outputs an electric signal corresponding to the stimulation response to the muscle relaxation monitoring apparatus 1. In the detection electrode section 20, in the case where the polarity of the detection electrode 20a is plus, for example, that of the detection electrode 20b is minus. In the detection electrode section 20, in the case where the observation portion is the adductor pollicis muscle, for example, the detection electrode 20a of the plus polarity is attached to the skin surface above the flexor tendon of, for example, the thumb that functions as a fixation portion, and the detection electrode 20b of the minus polarity is attached to the skin surface above the adductor pollicis muscle so as to enable the stimulation response (muscle response) of the adductor pollicis muscle to be detected. The detection electrode section 20 is connectable to and disconnectable from the muscle relaxation monitoring apparatus 1, and, when a failure or the like occurs, can be replaced with another one.

Then, the configuration of the muscle relaxation monitoring apparatus 1 will be described. The muscle relaxation monitoring apparatus 1 may include an inputting/ outputting section 2, a calibration processing section 3, a stimulation mode setting section 4, a stimulation generator 5, a response detector 6, an operating section 7, a controller 8, and a storage section 9.

When the electric signal that corresponds to the action potential of the muscle in the observation portion and detected by the detection electrode section 20 is input, the inputting/outputting section 2 outputs the signal to the calibration processing section 3 or the response detector 6. When the stimulation response value of a muscle that is obtained when the nerve in the observation portion is electrically stimulated in accordance with the stimulation mode is input from the response detector 6, the inputting/outputting section 2 outputs the stimulation response value to the patient monitor 100. As a result, the input stimulation response value is displayed on a screen of the patient monitor 100.

When a calibration mode is selected as an operation mode, the calibration processing section 3 performs a calibration process in which an electrical stimulation of a predetermined stimulation current value is performed on the subject, the stimulation current value according to the maximal stimulation of each subject is detected, and the stimulation current value according to the supramaximal stimulation of the subject is acquired based on the detected stimulation current value. In the calibration process, specifically, the calibration processing section 3 varies a stimulation current value from an initial stimulation current value that is the current value (initial value) at the beginning of the process, by a value corresponding to a predetermined step current value at each stimulation timing, and detects the amplitude peak value (the absolute value of the maximum displacement (the difference between the maximal value and the minimal value obtained after the detection timing of the maximal value) in one period) of an electric signal (action potential) due to a stimulation response of a muscle that is in the observation portion to which the electrical stimulation is applied. In the calibration process, the calibration processing section 3 further detects the stimulation current value of the maximal stimulation of the subject based on a result of a comparison (behavior of the peak value before and after the variation of the current value) of peak values before and after the variation of the detected step current value, and acquires a current value that is obtained by adding the value corresponding to the step current value to the detected stimulation current value, as the stimulation current value of the supramaximal stimulation of the subject. The acquired stimulation current value of the supramaximal stimulation is stored in the storage section 9.

When the calibration process is executed by the calibration processing section 3, the controller 8 controls the stimulation generator 5 and the response detector 6 to perform an electrical stimulating process using the stimulation current value that is set, a process of detecting an amplitude peak value that is based on a stimulation response of a muscle due to an electrical stimulation of the stimulation current value (hereinafter, such a peak value is referred to simply as "amplitude peak value based on the stimulation current value"), and like processes.

With respect to "initial stimulation current value" in the calibration process, an arbitrary current value is set by the user of the muscle relaxation monitoring apparatus 1, such as a medical person, and more specifically the current value is set based on information relating to the subject. The information relating to the subject may be set based on at least one of: the admission information of the subject that is input to the muscle relaxation monitoring apparatus 1, the patient monitor 100 connected to the muscle relaxation monitoring apparatus 1, or another medical apparatus; information of the subject that is measured and recorded in an medical apparatus; and the age, sex, and weight that are included in information that is determined when a medical person visually checks the subject; physiological information, disease information, and history information (visit history, surgery history, and the like) of the subject; event information; and like information (hereinafter, such information is referred to as "subject information"). In an example in which "initial stimulation current value" is set based on the subject information, in the case where the subject is determined to be an adult, the initial stimulation current value is set with reference to the vicinity of about 30 mA that is a mean value (mean stimulation current value) of the stimulation current value of the supramaximal stimulation in an adult, and set to a current value that is smaller than the maximum value of the stimulation current in the apparatus configuration (hereinafter, the current value is referred to as "maximal stimulation current value," and set to, for example, 60 mA). In the case where the subject is determined to be an infant or a neonatal infant, the initial stimulation current value may be set with reference to the vicinity of about 10 mA in order to reduce a physical damage. In the embodiment, as described above, the initial stimulation current value may be arbitrarily set in a range of, for example, 10 mA to 40 mA in accordance with the subject information. In the case where, even when the subject is an infant, for example, the subject has a weight of 20 kg or more, the initial stimulation current value may be set to about 30 mA.

In the muscle relaxation monitoring apparatus 1 of the embodiment, "initial stimulation current value" that is used in the calibration process is set to be smaller than the maximum value of the stimulation current in the apparatus configuration in accordance with the subject information. In the case where the subject is an adult, therefore, the process time period can be shortened as compared with the conventional calibration process in which the stimulation current value of the supramaximal stimulation is searched with starting from the maximal stimulation current value in the apparatus configuration. In the case where the subject is an infant or a neonatal infant, the initial stimulation current value is set in accordance with the subject, and therefore a physical damage is reduced as compared with the case where the detection is started from at least the maximum value (for example, 60 mA) of the stimulation current, and moreover the stimulation current value of the supramaximal stimulation can be detected in a short time period.

"Step current value" is set with reference to the initial stimulation current value in order to improve the speed of the calibration process, and enable the stimulation current value of the supramaximal stimulation to be easily acquired. In the case where the initial stimulation current value is set to "30 mA," for example, the step current value may be set to "3 mA" that is increased by 10% from the initial stimulation current value. When the step current value is set to be larger than the variable current value in the conventional calibration process, the speed of the calibration process can be improved. In the calibration process without requiring a cumbersome process, the stimulation current value of the supramaximal stimulation can be easily acquired, simply by adding (appending) a value corresponding to the step current value to the detected stimulation current value of the maximal stimulation.

"Stimulation timing" is set to, for example, 2 to 4 times per second (2 to 4 Hz). The stimulation timing is set so that the process time period can be shortened as compared with the conventional calibration process, and the stimulation current value of the supramaximal stimulation can be acquired from a stimulation current value that causes the correct maximal stimulation.

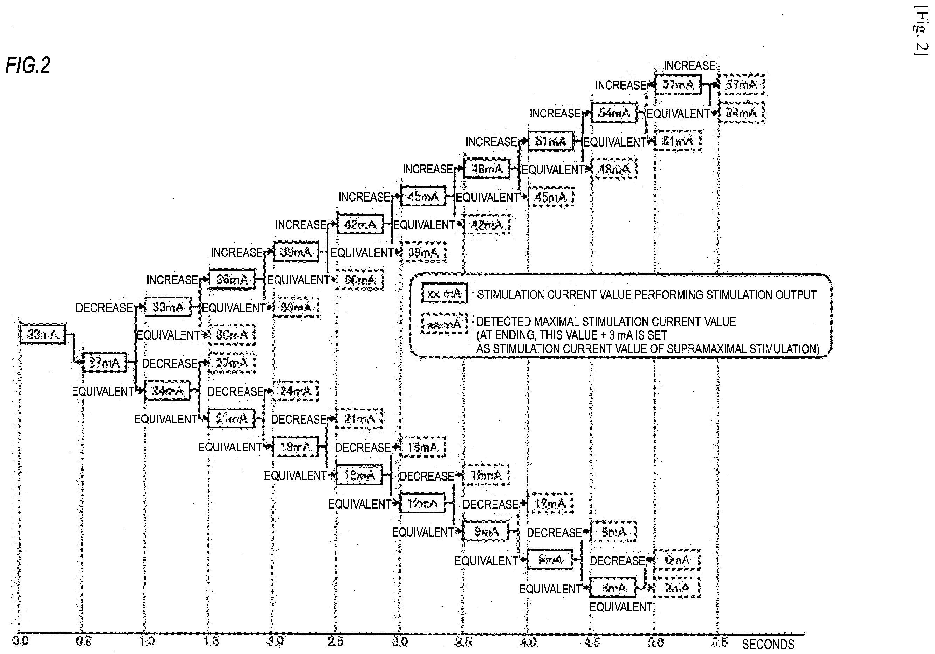

FIG. 2 conceptually illustrates the calibration process that is executed in the calibration processing section 3. The calibration process illustrated in FIG. 2 is an example in which the initial stimulation current value is set to "30 mA," the step current value to "3 mA," the stimulation timing to "2 Hz," the upper limit value of the stimulation current value of the supramaximal stimulation to "60 mA," and the lower limit value to "3 mA." The lateral direction of FIG. 2 coincides with the time axis, and indicates the time period elapsing from the starting time of the calibration process to each stimulation timing.

In the calibration process, "current value varying/comparing process" is executed in which the step current value is varied at each stimulation timing, and the amplitude peak values based on stimulation current values before and after the variation are compared with each other. The current value varying/comparing process includes two process modes of "current value increasing/comparing process" in which, while performing, as a current value variable process, a first current value variable process that increases by the step current value at each stimulation timing, the amplitude peak values based on stimulation current values before and after the increase of the step current value are compared with each other, and "current decreasing/comparing process" in which, while performing, as a current value variable process, a second current value variable process that decreases by the step current value at each stimulation timing, the amplitude peak values based on stimulation current values before and after the decrease of the step current value are compared with each other.

The process mode of the current value varying/comparing process is determined after the calibration process is started, and before the third stimulation timing (a timing after elapse of one second from the start of the process) in FIG. 2. When the process mode of the current value varying/comparing process is determined, a process of varying the stimulation current value, and that of comparing the peak values before and after the variation are thereafter sequentially performed at each stimulation timing in accordance with the determined process mode.

In FIG. 2, when "current value increasing/comparing process" is selected, at and after the third stimulation timing, the current value increasing/comparing process that is directed in the upper right direction in the figure is performed at each stimulation timing until the maximal stimulation of the subject is detected. When "current value decreasing/comparing process" is selected, at and after the third stimulation timing, the current value decreasing/comparing process that is directed in the lower right direction in the figure is performed at each stimulation timing until the maximal stimulation of the subject is detected.

As illustrated in FIG. 2, in the case where "current value increasing/comparing process" is selected as the current value varying/comparing process, the calibration processing section 3 detects the amplitude peak value that is based on the stimulation current value of "33 mA" which is obtained at the third stimulation timing by increasing the initial stimulation current value of "30 mA" by the value corresponding to the step current value. Next, the calibration processing section 3 compares the amplitude peak value based on the stimulation current value of "33 mA" that is increased by the value corresponding to the step current value, and the amplitude peak value based on the stimulation current value obtained before the increase by the value corresponding to the step current value (i.e., the amplitude peak value based on the initial stimulation current value of "30 mA"), with each other.

If it is determined that the amplitude peak value based on the stimulation current value of "33 mA" that is increased by the value corresponding to the step current value is increased from that based on the stimulation current value of "30 mA" obtained before the increase by the value corresponding to the step current value, the peak value is not yet saturated. Therefore, the calibration processing section 3 sets the stimulation current value to "36 mA" that is increased by the value corresponding to the step current value, and detects the amplitude peak value that is based on the stimulation current value.

Thereafter, the calibration processing section 3 sequentially increases by the step current value at each stimulation timing until the maximal stimulation of the subject is detected, compares the amplitude peak values that are based on the stimulation current values before and after the increase of the step current value, with each other, and performs the current value increasing/comparing process until the saturation of the peak value is confirmed. In the case where the peak value is not saturated when the stimulation current value reaches to 57 mA, "60 mA" that is the maximal stimulation current value is set as the stimulation current value of the supramaximal stimulation of the subject.

If it is determined that the amplitude peak value based on the stimulation current value of "33 mA" that is increased by the value corresponding to the step current value, and that based on the stimulation current value of "30 mA" obtained before the increase by the value corresponding to the step current value are equivalent to each other, the calibration processing section 3 determines that the peak value is saturated. Therefore, the calibration processing section 3 detects the stimulation current value of the maximal stimulation of the subject as the stimulation current value of "30 mA" obtained before the increase by the value corresponding to the step current value, and acquires "33 mA" that is obtained by adding the step current value to the stimulation current value of "30 mA," as the stimulation current value of the supramaximal stimulation of the subject.

In the case where "current value decreasing/comparing process" is selected as the current value varying/comparing process, the calibration processing section 3 detects the amplitude peak value that is based on the stimulation current value of "24 mA" which is obtained at the third stimulation timing by decreasing the stimulation current value of "27 mA" by the value corresponding to the step current value. Next, the calibration processing section 3 compares the amplitude peak value that is based on the stimulation current value of "27 mA" obtained before the decrease by the value corresponding to the step current value, and the amplitude peak value that is based on the stimulation current value of "24 mA" that is obtained by decreasing by the value corresponding to the step current value, with each other.

If it is determined that the amplitude peak value based on the stimulation current value of "24 mA" that is decreased by the value corresponding to the step current value is decreased from that based on the stimulation current value of "27 mA" obtained before the decrease by the value corresponding to the step current value, the calibration processing section 3 detects the stimulation current value of the maximal stimulation of the subject as the stimulation current value of "27 mA" obtained before the decrease by the value corresponding to the step current value. Then, the calibration processing section 3 acquires "30 mA" that is obtained by adding the step current value to the stimulation current value of "27 mA," as the stimulation current value of the supramaximal stimulation of the subject.

If it is determined that the amplitude peak value based on the stimulation current value of "24 mA" that is decreased by the value corresponding to the step current value, and that based on the stimulation current value of "27 mA" obtained before the decrease by the value corresponding to the step current value are equivalent to each other, the calibration processing section 3 determines that the peak value is saturated. Therefore, the calibration processing section 3 sets at the next stimulation timing the stimulation current value to "21 mA" obtained by decreasing by the value corresponding to the step current value, and detects the amplitude peak value that is based on the stimulation current value.

Thereafter, the calibration processing section 3 sequentially decreases by the value corresponding to the step current value at each stimulation timing until the maximal stimulation of the subject is detected, compares the amplitude peak values that are based on the stimulation current values before and after the decrease of the step current value, with each other, and performs the current value decreasing/comparing process until the decrease of the peak value is confirmed.

In the above-described calibration process, when the process mode of the current value varying/comparing process is to be determined, a process of detecting the amplitude peak value that is based on the stimulation current value of "27 mA" which is obtained at the second stimulation timing by decreasing the initial stimulation current value of "30 mA" by the step current value of "3 mA" is executed. This is because, as compared with the case where the stimulation current value is set to "33 mA" that is obtained by increasing the stimulation current value by the value corresponding to the step current value, a damage imposed on the subject is reduced, and the calibration process is safely executed. In the process of comparing the detected amplitude peak values, the determination of "equivalence" is conducted when the variation of the peak value is within a predetermined range (for example, 1% or smaller), and the determinations of "decrease" and "increase" are conducted when the variation of the peak value exceeds the predetermined range. When "equivalence" is determined, namely, it means that the two peak values that are comparison targets are saturated.

The stimulation mode setting section 4 performs a process in which one of a plurality of preset stimulation modes is appropriately changed to another one of the modes in accordance with the degree of progress of the muscle relaxation of the subject. Examples of the stimulation modes are "single stimulation mode", "Train-Of-Four simulation (TOF) mode", "Double Burst Stimulation (DBS) mode", "Tetanic stimulation (TET) mode", and "Post-Tetanic Count stimulation (PTC) mode". The stimulation mode setting section 4 sets an adequate stimulation mode in accordance with the degree of progress of the muscle relaxation, according to instructions from the controller 8.

The stimulation generator 5 is configured by an electric circuit for generating a stimulation pattern of a predetermined current value. The stimulation generator 5 performs a predetermined electrical stimulation (an electrical stimulation according to the stimulation current value that is set by the calibration processing section 3, or that according to the stimulation mode that is set by the stimulation mode setting section 4) on a peripheral nerve leading to the muscle of the observation portion of the subject, through the stimulation electrode section 10.

When the response detector 6 receives the electric signal that is based on the stimulation response of the muscle in the observation portion, through the inputting/ outputting section 2, the response detector acquires the stimulation response value that is based on the electric signal. When the muscle relaxation monitoring mode is selected as an operation mode, and the TOF mode is set as the stimulation mode, for example, the response detector 6 acquires, as the stimulation response, a ratio (ratio of a first stimulation and a fourth stimulation) of amplitude peak values in the electric signal according to the action potential of the muscle of the observation portion, as "TOF ratio," or the number of signals that appear in a predetermined time period, as "TOF count." The response detector 6 outputs the acquired stimulation response value to the patient monitor 100 through the inputting/outputting section 2.

When the calibration mode is selected as the operation mode, furthermore, the response detector 6 detects the amplitude peak value that is based on the electrical stimulation of the predetermined current value and performed by the stimulation generator 5, and outputs the peak value to the calibration processing section 3.

The operating section 7 is an interface that is attached to the case of the muscle relaxation monitoring apparatus 1, and that is used for performing various inputs to the muscle relaxation monitoring apparatus 1. When the operating section 7 is operated in order to select, as the operation mode, the calibration mode (the pre-muscle relaxation calibration mode or the post-muscle relaxation calibration mode) in which the calibration process is to be executed, or the muscle relaxation monitoring mode that is to be selected when a muscle relaxation monitoring process is to be executed in the stimulation mode, for example, the operation signal is output to the controller 8.

The controller 8 is configured by various processors such as a CPU (Central Processing Unit), a ROM, and a RAM. The controller 8 generally controls the sections constituting the muscle relaxation monitoring apparatus 1 while activating a predetermined process program based on various operation signals that are supplied from the operating section 7, to execute a predetermined process. When the operating section 7 is operated, and the calibration mode is selected as the operation mode, for example, the controller 8 appropriately controls the calibration processing section 3, the stimulation generator 5, and the response detector 6, and executes the calibration process according to the calibration mode.

The storage section 9 is an auxiliary storage device that stores various data, and stores various data that are necessary for driving the muscle relaxation monitoring apparatus 1, such as operation programs for the stimulation modes. Moreover, the stimulation current value of the supramaximal stimulation of the subject that is acquired in the calibration mode is written by the controller 8 in the storage section 9, and used in the case of the muscle relaxation monitoring mode.

### <Process operation>

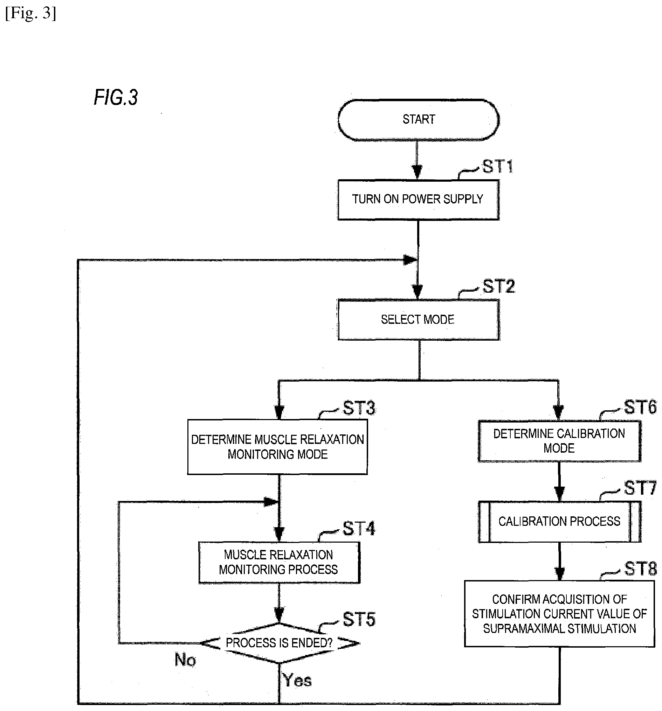

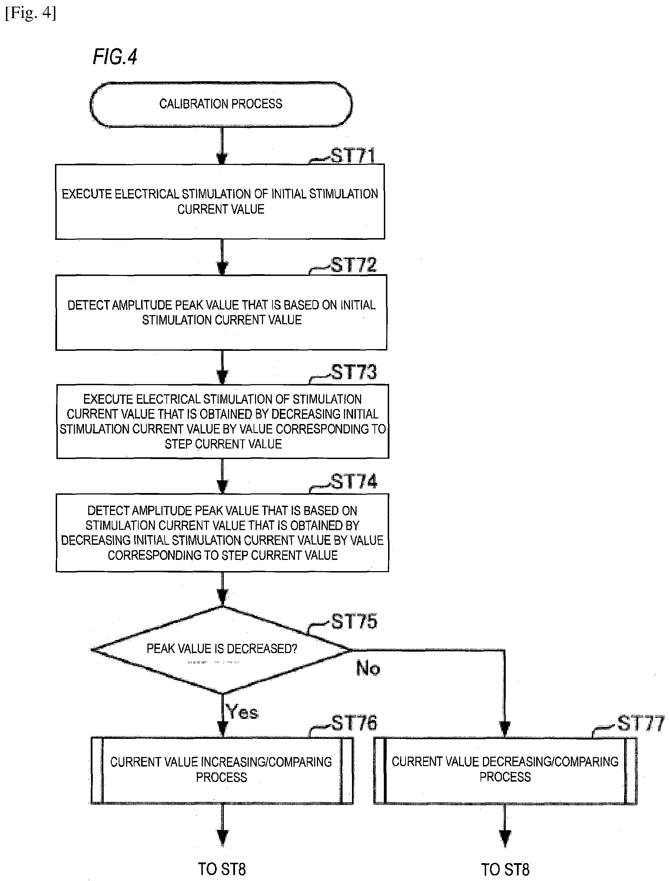

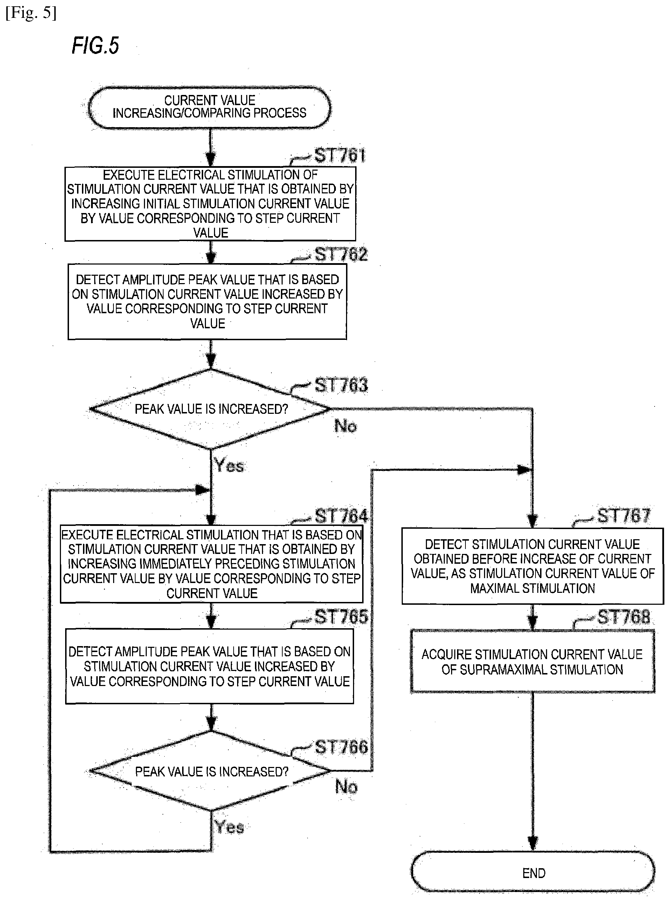

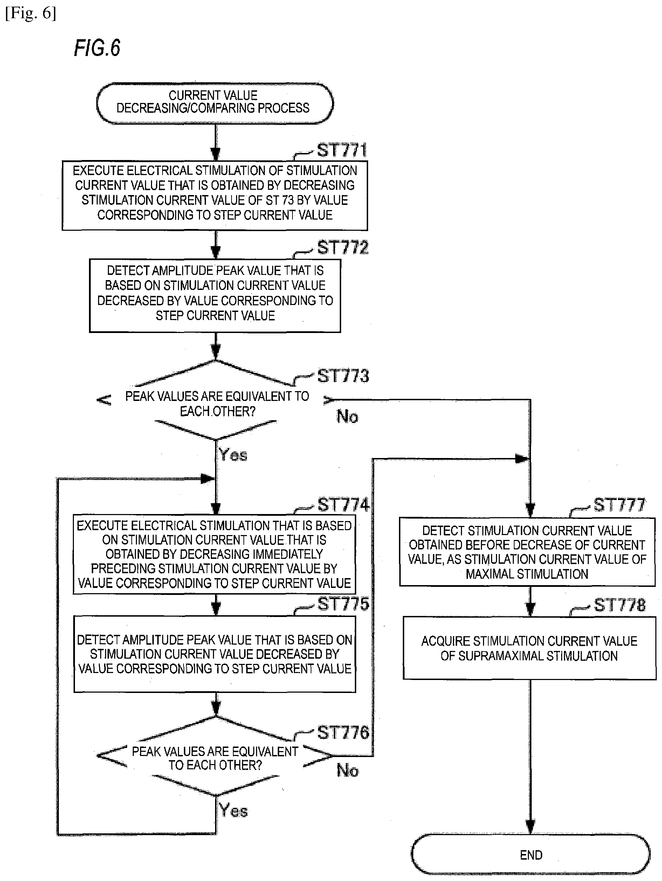

Referring to FIGS. 3 to 6, next, the process operation of the above-described muscle relaxation monitoring apparatus 1 will be described. FIG. 3 is a flowchart of a series of process operations of the muscle relaxation monitoring apparatus 1, FIG. 4 is a flowchart of process operations in the calibration process, FIG. 5 is a flowchart of the electric current value increasing/comparing process in the calibration process, and FIG. 6 is a flowchart of the electric current value decreasing/comparing process in the calibration process.

The operations that will be described indicate elements of steps in an exemplified order, and not limited to the presented specific order. Therefore, the flowcharts illustrated in FIGS. 3 to 6 can be exchanged in order as far as process results are consistent with each other.

### <Process of whole apparatus>

As illustrated in FIG. 3, in order to cause the muscle relaxation monitoring apparatus 1 to operate, the user turns on the power supply of the muscle relaxation monitoring apparatus 1 (ST1), and operates the operating section 7 to select the operation mode (ST2).

If, in ST2, "muscle relaxation monitoring mode" is selected and determined as the operation mode (ST3), the muscle relaxation monitoring apparatus 1 executes "muscle relaxation monitoring process" in which a process according to the muscle relaxation monitoring mode is performed to monitor the muscle relaxation state of the subject (ST4).

In the muscle relaxation monitoring process of ST4, the muscle relaxation monitoring apparatus 1 monitors the degree of progress of the muscle relaxation due to administration of the muscle relaxant agent to the subject, based on the stimulation response value that is produced by the electrical stimulation in the stimulation mode. In the monitoring of the muscle relaxation state, the controller 8 controls the stimulation mode setting section 4 so as to select an adequate stimulation mode according to the degree of progress of the muscle relaxation. In accordance with the selected stimulation mode, the controller 8 controls the stimulation generator 5 so as to electrically stimulate a peripheral nerve leading to a muscle in the observation portion. Then, the controller 8 controls the response detector 6 so as to acquire a stimulation response that is based on an electrical stimulation according to a stimulation mode such as the TOF ratio or the TOF count, and output stimulation response values according to these stimulation responses to the patient monitor 100 through the inputting/outputting section 2.

Then, the controller 8 determines whether the muscle relaxation monitoring process is ended or not (ST5). If it is determined that the muscle relaxation monitoring process is ended (ST5 - Yes), the process is ended, and the control again returns to ST2.

By contrast, if it is determined that the muscle relaxation monitoring process is not ended (ST5 - No), the control again returns to ST4, and the muscle relaxation monitoring process is continued.

If, in ST2, "calibration mode" is selected and determined as the operation mode (ST6), the muscle relaxation monitoring apparatus 1 executes "calibration process" in which a process according to the calibration mode is performed to acquire the stimulation current value of the supramaximal stimulation of the subject (ST7). "Calibration process" in ST7 will be described in detail later with reference to FIG. 4.

When, then, the controller 8 confirms that the stimulation current value of the supramaximal stimulation of the subject is acquired in the calibration process (ST8), the control again returns to ST2. After the calibration process, usually, the muscle relaxation monitoring mode is selected by the user as the operation mode, and the control is transferred to the muscle relaxation monitoring process for the subject.

### <Calibration process>

Next, a series of operations of the calibration process in the calibration mode will be described with reference to FIG. 4.

As illustrated in FIG. 4, when the calibration process is started, the calibration processing section 3 performs an electrical stimulation of the initial stimulation current value (ST71), and detects an amplitude peak value that is based on the initial stimulation current value (ST72). The electrical stimulation of the initial stimulation current value in ST71 functions as a trigger for starting the calibration process. Next, the calibration processing section 3 performs, at the next stimulation timing, an electrical stimulation of a stimulation current value that is obtained by decreasing the initial stimulation current value by a value corresponding to the step current value (ST73), and detects the amplitude peak value at this timing (ST74).

Then, the calibration processing section 3 compares the amplitude peak value that is based on the stimulation current value obtained before the decrease by the value corresponding to the step current value (i.e., the amplitude peak value acquired in ST72), and the amplitude peak value that is based on the stimulation current value that is obtained by decreasing by the value corresponding to the step current value (i.e., the amplitude peak value acquired in ST74), with each other, and determines whether the peak value is decreased by the decrease of the value corresponding to the step current value or not (ST75).

If, in ST75, it is determined that the amplitude peak value based on the stimulation current value after the variation of the current value is decreased from that based on the stimulation current value before the variation of the current value (ST75 - Yes), the calibration processing section 3 executes, in a subsequent process, "current value increasing/comparing process" (ST76). "Current value increasing/comparing process" in ST76 will be described in detail later with reference to FIG. 5.

By contrast, if, in ST75, it is determined that the amplitude peak value based on the stimulation current value before the variation of the current value, and that based on the stimulation current value after the variation of the current value are equivalent to each other, or that the amplitude peak value after the variation of the current value is increased (ST75 - No), "Current value decreasing/comparing process" (ST77) is executed in the subsequent process. "Current value decreasing/comparing process" in ST77 will be described in detail later with reference to FIG. 6.

When the stimulation current value that causes the supramaximal stimulation of the subject is acquired in ST76 or ST77, then, the control proceeds to ST8 of FIG. 3.

### <Current value increasing/comparing process>

Next, the current value increasing/comparing process in the calibration process will be described with reference to FIG. 5. In the current value increasing/comparing process, the initial stimulation current value is set as the initial value, and, while increasing by the value corresponding to the step current value at each stimulation timing, the stimulation current value of the supramaximal stimulation of the subject is acquired based on the behavior of the amplitude peak value based on the electrical stimulations before and after the increase of the current value.

In the current value increasing/comparing process, the calibration processing section 3 performs, at a predetermined stimulation timing, an electrical stimulation of a stimulation current value that is obtained by increasing the initial stimulation current value by the value corresponding to the step current value (ST761). Next, the calibration processing section 3 detects an amplitude peak value that is based on the stimulation current value by which the electrical stimulation is performed in ST761 (ST762), compares the amplitude peak value that is based on the stimulation current value obtained before the increase by the value corresponding to the step current value (i.e., the amplitude peak value acquired in ST72 of FIG. 4), and the amplitude peak value that is based on the stimulation current value that is obtained by increasing by the value corresponding to the step current value (i.e., the amplitude peak value acquired in ST762), with each other, and determines whether the peak value is increased by the increase of the value corresponding to the step current value or not (ST763).

If, in ST763, the calibration processing section 3 determines that the amplitude peak value that is based on the stimulation current value that is obtained by increasing by the value corresponding to the step current value is increased from the amplitude peak value that is based on the stimulation current value obtained before the increase by the value corresponding to the step current value (ST763 - Yes), the peak value is not yet saturated, an electrical stimulation is therefore performed by a stimulation current value that is obtained by further increasing the immediately preceding stimulation current value by the value corresponding to the step current value (ST764), and detects an amplitude peak value that is based on the stimulation current value (ST765). Then, the calibration processing section 3 compares the amplitude peak value that is based on the stimulation current value obtained before the increase by the value corresponding to the step current value, and the amplitude peak value that is based on the stimulation current value that is obtained by increasing by the value corresponding to the step current value, with each other, and determines whether the peak values are increased or not (ST766).

By contrast, if, in ST763, the calibration processing section 3 determines that the amplitude peak value that is based on the stimulation current value that is obtained by increasing by the value corresponding to the step current value, and the amplitude peak value that is based on the stimulation current value obtained before the increase by the value corresponding to the step current value are equivalent to each other (ST763 - No), the peak value is saturated, and therefore the stimulation current value obtained before the increase by the value corresponding to the step current value is detected as the stimulation current value of the maximal stimulation of the subject (ST767). Then, the calibration processing section 3 acquires a stimulation current value that is obtained by adding the value corresponding to the step current value to the stimulation current value of the maximal stimulation, as the stimulation current value of the supramaximal stimulation of the subject (ST768), and the process is ended.

If, in ST766, the calibration processing section 3 determines that the amplitude peak value that is based on the stimulation current value that is obtained by increasing by the value corresponding to the step current value is increased from the amplitude peak value that is based on the stimulation current value obtained before the increase by the value corresponding to the step current value (ST766 - Yes), the peak value is not yet saturated, the control therefore returns to ST764, and, at the next stimulation timing, an electrical stimulation is performed by a stimulation current value that is obtained by further increasing the immediately preceding stimulation current value by the value corresponding to the step current value.

The processes of ST764 to ST766 are repeatedly performed until the stimulation current value that causes the maximal stimulation of the subject is detected.

By contrast, if, in ST766, the calibration processing section 3 determines that the amplitude peak value that is based on the stimulation current value that is obtained by increasing by the value corresponding to the step current value, and the amplitude peak value that is based on the stimulation current value obtained before the increase by the value corresponding to the step current value are equivalent to each other (ST766 - No), the processes of ST767 and ST768 are sequentially performed, and the stimulation current value that causes the supramaximal stimulation of the subject is acquired.

### <Current value decreasing/comparing process>

Next, the current value decreasing/comparing process in the calibration process will be described with reference to FIG. 6. In the current value decreasing/comparing process, the stimulation current value that is obtained by decreasing the initial stimulation current value by the value corresponding to the step current value is set as the initial value, and, while decreasing by the value corresponding to the step current value at each stimulation timing, the stimulation current value of the supramaximal stimulation of the subject is acquired based on the behavior of the amplitude peak value based on the electrical stimulations before and after the decrease of the current value.

In the current value decreasing/comparing process, the calibration processing section 3 performs, at a predetermined stimulation timing, an electrical stimulation of a stimulation current value that is obtained by decreasing the initial stimulation current value by which the electrical stimulation is performed in ST73 of FIG. 4, by the value corresponding to the step current value (ST771). Next, the calibration processing section 3 detects an amplitude peak value that is based on the stimulation current value by which the electrical stimulation is performed in ST771 (ST772). Then, the calibration processing section 3 compares the amplitude peak value that is based on the stimulation current value obtained before the decrease by the value corresponding to the step current value (i.e., the amplitude peak value acquired in ST73 of FIG. 4), and the amplitude peak value that is based on the stimulation current value that is obtained by decreasing by the value corresponding to the step current value (i.e., the amplitude peak value acquired in ST772), with each other, and determines whether the peak values are made equivalent to each other by the decrease of the value corresponding to the step current value or not (ST773).

If, in ST773, the calibration processing section 3 determines that the amplitude peak value that is based on the stimulation current value that is obtained by decreasing by the value corresponding to the step current value, and the amplitude peak value that is based on the stimulation current value obtained before the decrease by the value corresponding to the step current value are equivalent to each other (ST773 - Yes), it is determined that the peak value is still saturated. Therefore, the calibration processing section 3 performs, at the next stimulation timing, an electrical stimulation of a stimulation current value that is obtained by further decreasing the immediately preceding stimulation current value by the value corresponding to the step current value (ST774), and detects an amplitude peak value that is based on the stimulation current value (ST775). Then, the calibration processing section 3 compares the amplitude peak value that is based on the stimulation current value obtained before the decrease by the value corresponding to the step current value, and the amplitude peak value that is based on the stimulation current value that is obtained by decreasing by the value corresponding to the step current value, with each other, and determines whether the peak values are equivalent to each other or not (ST776).

By contrast, if, in ST773, it is determined that the amplitude peak value that is based on the stimulation current value that is obtained by decreasing by the value corresponding to the step current value is decreased from the amplitude peak value that is based on the stimulation current value obtained before the decrease by the value corresponding to the step current value (ST773 - No), the calibration processing section 3 detects the stimulation current value obtained before the decrease by the value corresponding to the step current value, as the stimulation current value of the maximal stimulation of the subject (ST777). Then, the calibration processing section 3 acquires a stimulation current value that is obtained by adding the value corresponding to the step current value to the stimulation current value of the maximal stimulation, as the stimulation current value of the supramaximal stimulation of the subject (ST778), and the process is ended.

If, in ST776, the calibration processing section 3 determines that the amplitude peak value that is based on the stimulation current value that is obtained by decreasing by the value corresponding to the step current value is equivalent to the amplitude peak value that is based on the stimulation current value obtained before the decrease by the value corresponding to the step current value (ST776 - Yes), the peak value is still saturated, the control therefore returns to ST774, and, at the next stimulation timing, an electrical stimulation is performed by a stimulation current value that is obtained by decreasing the immediately preceding stimulation current value by the value corresponding to the step current value.

The processes of ST774 to ST776 are repeatedly performed until the stimulation current value that causes the maximal stimulation of the subject is detected.

By contrast, if, in ST776, the calibration processing section 3 determines that the amplitude peak value that is based on the stimulation current value that is obtained by decreasing by the value corresponding to the step current value is decreased from the amplitude peak value that is based on the stimulation current value obtained before the decrease by the value corresponding to the step current value (ST776 - No), the processes of ST777 and ST778 are sequentially performed, and the stimulation current value that causes the supramaximal stimulation of the subject is acquired.

### <Function and Effects>

As described above, the muscle relaxation monitoring apparatus 1 of the embodiment includes the calibration processing section 3 for acquiring the stimulation current value of the supramaximal stimulation exceeding a maximal stimulation of the muscle which is the observation portion of the subject. The calibration processing section 3 sets the initial stimulation current value that is set to be smaller than the maximum value of the stimulation current, as a starting stimulation current value, determines, as a current value variable process, one of: a first current value variable process that increases by a value corresponding to a predetermined step current value at a predetermined stimulation timing; and a second current value variable process that decreases by the value corresponding to the step current value at the stimulation timing, detects, as a first peak value, an amplitude peak value of an electric signal that is based on a stimulation response of the muscle due to an electrical stimulation of a first stimulation current value that is a stimulation current value obtained before the stimulation current value is varied by the value corresponding to the step current value in the determined current value variable process, detects, as a second peak value, an amplitude peak value of an electric signal that is based on a stimulation response of the muscle due to an electrical stimulation of a second stimulation current value that is varied by the value corresponding to the step current value from the first stimulation current value, based on the determined current value variable process, at a stimulation timing that is next to the stimulation timing of the first stimulation current value, detects a stimulation current value of a maximal stimulation of the subject, based on a result of a comparison of the first peak value and the second peak value, and acquires a stimulation current value that is obtained by adding the value corresponding to the step current value to the stimulation current value of the maximal stimulation of the subject, as the stimulation current value of the supramaximal stimulation of the subject.

In the apparatus, the initial stimulation current value that is used in the starting of the calibration process is set to a current value that is smaller than the maximum value of the stimulation current (the maximal stimulation current in the apparatus configuration), and therefore the stimulation current value of the supramaximal stimulation can be acquired in a short time period while reducing the burden imposed on the subject.

In the muscle relaxation monitoring apparatus 1 of the embodiment, the initial stimulation current value is from 10 mA to smaller than 60 mA, and set based on information relating to the subject (subject information).

According to the configuration, in the case where the subject is an adult, the process time period can be shortened as compared with the conventional calibration process in which the stimulation current value of the supramaximal stimulation is searched with starting from the maximal stimulation current value that causes the stimulation current value to be maximum. In the case where the subject is an infant or a neonatal infant, a physical damage is reduced as compared with the case where the detection is started from the maximal stimulation current that causes the stimulation current value to be maximum, and moreover the stimulation current value of the supramaximal stimulation can be detected in a short time period.

In the muscle relaxation monitoring apparatus 1 of the embodiment, the calibration processing section determines one of the first current value variable process and the second current value variable process based on a result of a comparison of the amplitude peak value of the electric signal that is based on the stimulation response of the muscle due to the electrical stimulation of the initial stimulation current value, and that of the electric signal that is based on the stimulation response of the muscle due to the electrical stimulation of the stimulation current value that is obtained by decreasing the initial stimulation current value by the value corresponding to the step current value.

At the stimulation timing, therefore, the current value increasing/comparing process in which an increase in the step current value is performed, and the current value decreasing/comparing process in which a decrease in the step current value is performed can be adequately set for each subject, and hence the stimulation current value of the supramaximal stimulation of the subject in the calibration processing section can be acquired in a short time period.

In the muscle relaxation monitoring apparatus 1 of the embodiment, the step current value is set based on the initial stimulation current value.

In the calibration process, when the stimulation current value of the maximal stimulation of the subject is acquired, therefore, a stimulation current value that is obtained simply by increasing by the value corresponding to the step current value can be acquired as the stimulation current value of the supramaximal stimulation of the subject.

In the muscle relaxation monitoring apparatus 1 of the embodiment, the stimulation timing is set in the range of 2 to 4 Hz.

According to the configuration, the interval of stimulation timings is shorter as compared with the conventional calibration process, and therefore the stimulation current value of the supramaximal stimulation of the subject can be acquired in a shorter time period.

In the muscle relaxation monitoring apparatus 1 of the embodiment, in the case where the current value increasing/comparing process is executed in which the first peak value that is based on the first stimulation current value that is a stimulation current value obtained before the stimulation current value is increased, and the second peak value that is based on the second stimulation current value that is obtained by increasing the first stimulation current value by the value corresponding to the step current value based on the first current value variable process are compared with each other, when it is determined that the first peak value and the second peak value are equivalent to each other, the calibration processing section 3 detects the first stimulation current value as the stimulation current value of the maximal stimulation. In the case where the current value decreasing/comparing process is executed in which the first peak value that is based on the first stimulation current value that is a stimulation current value obtained before the stimulation current value is decreased, and the second peak value that is based on the second stimulation current value that is obtained by decreasing the first stimulation current value by the value corresponding to the step current value based on the second current value variable process are compared with each other, when it is determined that the second peak value is decreased from the first peak value, the calibration processing section 3 detects the first stimulation current value as the stimulation current value of the maximal stimulation.

According to the configuration, the stimulation current value in a state where the peak current is saturated can be known, and therefore an adequate stimulation current value can be detected as the stimulation current value of the maximal stimulation of the subject.

The muscle relaxation monitoring apparatus 1 of the embodiment has the configuration in which the calibration processing section 3 is disposed in an electromyography that monitors the muscle relaxation state of the subject based on the action potential of muscle fibers of a muscle that is induced when an electrical stimulation is applied to a nerve.

Even in the case where an electromyography is used as the muscle relaxation monitoring apparatus 1, the initial stimulation current value that is used in the starting of the calibration process is set to be smaller than the maximum value of the stimulation current, and therefore the stimulation current value of the supramaximal stimulation can be acquired in a short time period while reducing the burden imposed on the subject.

The calibration processing method of the embodiment includes: a process of determining, as a current value variable process, one of: a first current value variable process that increases by a value corresponding to a predetermined step current value at a predetermined stimulation timing; and a second current value variable process that decreases by the value corresponding to the step current value at the stimulation timing; a process of setting an initial stimulation current value that is set to be smaller than a maximum value of a stimulation current value, as a starting stimulation current value, and detecting, as a first peak value, an amplitude peak value of an electric signal that is based on a stimulation response of a muscle due to an electrical stimulation of a first stimulation current value that is a stimulation current value obtained before the stimulation current value is varied by the value corresponding to the step current value in the determined current value variable process; a process of detecting, as a second peak value, an amplitude peak value of an electric signal that is based on a stimulation response of the muscle due to an electrical stimulation of a second stimulation current value that is varied by the value corresponding to the step current value from the first stimulation current value, based on the determined current value variable process, at a stimulation timing that is next to the stimulation timing of the first stimulation current value; and a process of detecting a stimulation current value of a maximal stimulation of the subject, based on a result of a comparison of the first peak value and the second peak value, and acquiring a stimulation current value that is obtained by adding the value corresponding to the step current value to the stimulation current value of the maximal stimulation of the subject, as the stimulation current value of the supramaximal stimulation of the subject.

In the method, the initial stimulation current value is set to be smaller than the maximum value of the stimulation current, and therefore the stimulation current value of the supramaximal stimulation can be acquired in a short time period while reducing the burden imposed on the subject.

Although, in the above description, the embodiment in which an electromyography that monitors the muscle relaxation state or the awake state, based on the myopotential of a subject is used as the muscle relaxation monitoring apparatus 1 has been described, the type of the monitor is not particularly limited as far as the muscle relaxation monitor performs a calibration process for acquiring the stimulation current value of the supramaximal stimulation of the subject. Another mode of the muscle relaxation monitoring apparatus 1 may be, for example, an apparatus that sequentially observes the muscle contraction state of a muscle which is the observation portion, and can objectively monitor the muscle relaxation state of the subject, such as an ac-celeromyography (AMG) that uses an acceleration transducer.

In the above-described embodiment, the inputting/outputting section 2, calibration processing section 3, stimulation mode setting section 4, stimulation generator 5, response detector 6, operating section 7, controller 8, and storage section 9 of the muscle relaxation monitoring apparatus 1 are disposed in the muscle relaxation monitoring apparatus 1. However, the configuration is not limited to this. The above-described configuration or a part of the configuration may be disposed in the patient monitor 100 connected to the muscle relaxation monitoring apparatus 1, another medical apparatus, or the like.

Although, in the above-described embodiment, the method of setting the initial stimulation current value is described by the configuration where the user of the apparatus, such as a medical person sets the initial stimulation current value based on the subject information, an initial stimulation current value that is adequate to the subject may be automatically set based on, for example, the subject information.

Although, in the above-described embodiment, the initial stimulation current value is a current value that is smaller than a stimulation current value (for example, 60 mA) that is maximum in the preset apparatus configuration, the initial stimulation current value is not limited to a current value that is maximum in the apparatus configuration, and may be a current value that is smaller than a stimulation current value that is maximum in a range of a stimulation current value which is arbitrarily set by the user or on the side of the apparatus.

In the above-described embodiment, the inputting/outputting section 2 of the muscle relaxation monitoring apparatus 1 has the mode where the stimulation response value acquired by the muscle relaxation monitoring apparatus 1 is displayed on the screen of the patient monitor 100 that is the connection destination. However, the mode is not limited to this. A configuration in which the muscle relaxation monitoring apparatus 1 includes a displaying section that can display the stimulation response value may be employed as the display destination of the stimulation response value. Moreover, the display destination of the stimulation response value may be a displaying apparatus that is separated from the muscle relaxation monitoring apparatus 1 (for example, a displaying apparatus such as an organic or inorganic EL display, or a liquid crystal display, or a portable terminal such as a smartphone or a tablet terminal).

This application claims priority to Japanese Patent Application No. 2019-198590 filed on October 31, 2019.

### Industrial Applicability

According to the present disclosed subject matter, there is provided a muscle relaxation monitoring apparatus and a calibration processing method in which it is possible to, while reducing the burden imposed on a subject, detect a stimulation current value that causes the supramaximal stimulation according to the subject, in a short time period.

## Claims

1. A muscle relaxation monitoring apparatus comprising
a calibration processing section for acquiring a stimulation current value of a supramaximal stimulation exceeding a maximal stimulation of a muscle of a subject,
wherein the calibration processing section is configured to:
set (ST71) an initial stimulation current value that is set to be smaller than a maximum value of a stimulation current in a configuration of the apparatus in accordance with subject information, as a starting stimulation current value;
determine (ST75), as a current value variable process, one of: a first current value variable process (ST76) that increases by a value corresponding to a predetermined step current value at a predetermined stimulation timing; and a second current value variable process (ST77) that decreases by the value corresponding to the step current value at the stimulation timing, the determination (ST75) being based on a result of a comparison of an amplitude peak value of the electric signal that is based on the stimulation response of the muscle due to the electrical stimulation of the initial stimulation current value, and an amplitude peak value of the electric signal that is based on the stimulation response of the muscle due to the electrical stimulation of the stimulation current value that is obtained by decreasing the initial stimulation current value by the value corresponding to the step current value;
detect (ST72), as a first peak value, an amplitude peak value of an electric signal that is based on a stimulation response of the muscle due to an electrical stimulation of a first stimulation current value that is a stimulation current value obtained before the stimulation current value is varied by the value corresponding to the step current value in the determined current value variable process;
detect (ST765, ST775), as a second peak value, an amplitude peak value of an electric signal that is based on a stimulation response of the muscle due to an electrical stimulation of a second stimulation current value that is varied by the value corresponding to the step current value from the first stimulation current value, based on the determined current value variable process, at a stimulation timing that is next to the stimulation timing of the first stimulation current value; and
detect (ST767, ST777) a stimulation current value of a maximal stimulation of the muscle of the subject, based on a result of a comparison of the first peak value and the second peak value, and acquire (ST768, ST778) a stimulation current value that is obtained by adding the step current value to the stimulation current value of the maximal stimulation of the subject, as the stimulation current value of the supramaximal stimulation of the subject.

2. The muscle relaxation monitoring apparatus according to claim 1, wherein the initial stimulation current value is a value that is from 10 mA to smaller than 60 mA.

3. The muscle relaxation monitoring apparatus according to claim 1 or 2, wherein the initial stimulation current value is set based on information relating to the subject.

4. The muscle relaxation monitoring apparatus according to any one of claims 1 to 3, wherein the step current value is set based on the initial stimulation current value.

5. The muscle relaxation monitoring apparatus according to any one of claims 1 to 4, wherein the stimulation timing is set in a range of 2 to 4 Hz.

6. The muscle relaxation monitoring apparatus according to any one of claims 1, 4 and 5, wherein,
in a case where a current value increasing/comparing process is executed in which the first peak value that is based on the first stimulation current value that is a stimulation current value obtained before the stimulation current value is increased, and the second peak value that is based on the second stimulation current value that is obtained by increasing the first stimulation current value by the value corresponding to the step current value based on the first current value variable process are compared (ST766) with each other,
when it is determined that the first peak value and the second peak value are equivalent to each other, the calibration processing section detects (ST767) the first stimulation current value as the stimulation current value of the maximal stimulation.

7. The muscle relaxation monitoring apparatus according to any one of claims 1 and 4 to 6, wherein,
in a case where a current value decreasing/comparing process is executed in which the first peak value that is based on the first stimulation current value that is a stimulation current value obtained before the stimulation current value is decreased, and the second peak value that is based on the second stimulation current value that is obtained by decreasing the first stimulation current value by the value corresponding to the step current value based on the second current value variable process are compared (ST776) with each other,
when it is determined that the second peak value is decreased from the first peak value, the calibration processing section detects (ST777) the first stimulation current value as the stimulation current value of the maximal stimulation.

8. The muscle relaxation monitoring apparatus according to any one of claims 1 to 7, wherein
the apparatus has a configuration in which
the calibration processing section is disposed in an electromyography that monitors a muscle relaxation state of the subject based on an action potential of muscle fibers of a muscle that is induced when an electrical stimulation is applied to a nerve of the subject.

9. The muscle relaxation monitoring apparatus according to claim 1, wherein the current value variable process includes two process modes of a current value increasing process and a current value decreasing process.

10. The muscle relaxation monitoring apparatus according to claim 1, wherein the predetermined stimulation timing includes 0.5 seconds.

11. A calibration processing method for acquiring a stimulation current value of a supramaximal stimulation exceeding a maximal stimulation of a muscle of a subject, wherein
the method comprises:
a process of determining (ST75), as a current value variable process, one of: a first current value variable process (ST76) that increases by a value corresponding to a predetermined step current value at a predetermined stimulation timing; and a second current value variable process (ST77) that decreases by the value corresponding to the step current value at the stimulation timing, the determination (ST75) being based on a result of a comparison of an amplitude peak value of the electric signal that is based on the stimulation response of the muscle due to the electrical stimulation of the initial stimulation current value, and an amplitude peak value of the electric signal that is based on the stimulation response of the muscle due to the electrical stimulation of the stimulation current value that is obtained by decreasing the initial stimulation current value by the value corresponding to the step current value;
a process of setting (ST71) an initial stimulation current value that is set to be smaller than a maximum value of a stimulation current value in an apparatus configuration in accordance with subject information, as a starting stimulation current value, and detecting (ST762, ST772), as a first peak value, an amplitude peak value of an electric signal that is based on a stimulation response of the muscle due to an electrical stimulation of a first stimulation current value that is a stimulation current value obtained before the stimulation current value is varied by the value corresponding to the step current value in the determined current value variable process;
a process of detecting (ST72), as a second peak value, an amplitude peak value of an electric signal that is based on a stimulation response of the muscle due to an electrical stimulation of a second stimulation current value that is varied by the value corresponding to the step current value from the first stimulation current value, based on the determined current value variable process, at a stimulation timing that is next to the stimulation timing of the first stimulation current value; and
a process of detecting (ST767, ST777) a stimulation current value of a maximal stimulation of the muscle of the subject, based on a result of a comparison of the first peak value and the second peak value, and acquiring (ST768, 778) a stimulation current value that is obtained by adding the step current value to the stimulation current value of the maximal stimulation of the subject, as the stimulation current value of the supramaximal stimulation of the subject.

12. The calibration processing method according to claim 11, wherein the current value variable process includes two process modes of a current value increasing process and a current value decreasing process.

13. The calibration processing method according to claim 11, wherein the predetermined stimulation timing includes 0.5 seconds.

## Patentansprüche

1. Vorrichtung zur Überwachung der Muskelentspannung, umfassend
einen Kalibrierungsverarbeitungsabschnitt zum Ermitteln eines Stimulationsstromwerts einer supramaximalen Stimulation, die eine maximale Stimulation eines Muskels einer Person überschreitet,
wobei der Kalibrierungsverarbeitungsabschnitt dazu eingerichtet ist:
(ST71) einen anfänglichen Stimulationsstromwert, der so eingestellt ist, dass er kleiner als ein Maximalwert eines Stimulationsstroms in einer Konfiguration der Vorrichtung gemäß Informationen über die Person ist, als einen Ausgangsstimulationsstromwert einzustellen;
(ST75) als einen Stromwertänderungsprozess einen der folgenden Prozesse zu bestimmen: einen ersten Stromwertänderungsprozess (ST76), der zu einem vorbestimmten Stimulationszeitpunkt um einen einem vorbestimmten Stromschrittwert entsprechenden Wert erhöht; und einen zweiten Stromwertänderungsprozess (ST77), der zu dem Stimulationszeitpunkt um den dem Stromschrittwert entsprechenden Wert verringert, wobei die Bestimmung (ST75) auf einem Ergebnis eines Vergleichs eines Amplitudenspitzenwerts des elektrischen Signals, das auf der Stimulationsantwort des Muskels infolge der elektrischen Stimulation mit dem anfänglichen Stimulationsstromwert beruht, und eines Amplitudenspitzenwerts des elektrischen Signals beruht, das auf der Stimulationsantwort des Muskels infolge der elektrischen Stimulation mit dem Stimulationsstromwert beruht, der durch Verringern des anfänglichen Stimulationsstromwerts um den dem Stromschrittwert entsprechenden Wert erhalten wird;
(ST72) als einen ersten Spitzenwert einen Amplitudenspitzenwert eines elektrischen Signals zu erfassen, das auf einer Stimulationsantwort des Muskels infolge einer elektrischen Stimulation mit einem ersten Stimulationsstromwert beruht, bei dem es sich um einen Stimulationsstromwert handelt, der erhalten wird, bevor der Stimulationsstromwert in dem bestimmten Stromwertänderungsprozess um den dem Stromschrittwert entsprechenden Wert geändert wird;
(ST765, ST775) als einen zweiten Spitzenwert einen Amplitudenspitzenwert eines elektrischen Signals zu erfassen, das auf einer Stimulationsantwort des Muskels infolge einer elektrischen Stimulation mit einem zweiten Stimulationsstromwert beruht, der auf Grundlage des bestimmten Stromwertänderungsprozesses um den dem Stromschrittwert entsprechenden Wert gegenüber dem ersten Stimulationsstromwert geändert wird, und zwar zu einem Stimulationszeitpunkt, der auf den Stimulationszeitpunkt des ersten Stimulationsstromwerts folgt; und
(ST767, ST777) auf Grundlage eines Ergebnisses eines Vergleichs des ersten Spitzenwerts und des zweiten Spitzenwerts einen Stimulationsstromwert einer maximalen Stimulation des Muskels der Person zu erfassen und (ST768, ST778) einen Stimulationsstromwert, der durch Addieren des Stromschrittwerts zu dem Stimulationsstromwert der maximalen Stimulation der Person erhalten wird, als den Stimulationsstromwert der supramaximalen Stimulation der Person zu ermitteln.

2. Vorrichtung zur Überwachung der Muskelentspannung nach Anspruch 1, wobei der anfängliche Stimulationsstromwert ein Wert von 10 mA bis kleiner als 60 mA ist.

3. Vorrichtung zur Überwachung der Muskelentspannung nach Anspruch 1 oder 2, wobei der anfängliche Stimulationsstromwert auf Grundlage von Informationen über die Person eingestellt wird.

4. Vorrichtung zur Überwachung der Muskelentspannung nach einem der Ansprüche 1 bis 3, wobei der Stromschrittwert auf Grundlage des anfänglichen Stimulationsstromwerts eingestellt wird.

5. Vorrichtung zur Überwachung der Muskelentspannung nach einem der Ansprüche 1 bis 4, wobei der Stimulationszeitpunkt in einem Bereich von 2 bis 4 Hz eingestellt wird.

6. Vorrichtung zur Überwachung der Muskelentspannung nach einem der Ansprüche 1, 4 und 5, wobei,
in einem Fall, in dem ein Stromwerterhöhungs-/Vergleichsprozess ausgeführt wird, bei dem der erste Spitzenwert, der auf dem ersten Stimulationsstromwert beruht, bei dem es sich um einen Stimulationsstromwert handelt, der erhalten wird, bevor der Stimulationsstromwert erhöht wird, und der zweite Spitzenwert, der auf dem zweiten Stimulationsstromwert beruht, der durch Erhöhen des ersten Stimulationsstromwerts um den dem Stromschrittwert entsprechenden Wert auf Grundlage des ersten Stromwertänderungsprozesses erhalten wird, miteinander verglichen werden (ST766),
wenn bestimmt wird, dass der erste Spitzenwert und der zweite Spitzenwert einander gleichwertig sind, der Kalibrierungsverarbeitungsabschnitt (ST767) den ersten Stimulationsstromwert als den Stimulationsstromwert der maximalen Stimulation erfasst.

7. Vorrichtung zur Überwachung der Muskelentspannung nach einem der Ansprüche 1 und 4 bis 6, wobei,
in einem Fall, in dem ein Stromwertverringerungs-/Vergleichsprozess ausgeführt wird, bei dem der erste Spitzenwert, der auf dem ersten Stimulationsstromwert beruht, bei dem es sich um einen Stimulationsstromwert handelt, der erhalten wird, bevor der Stimulationsstromwert verringert wird, und der zweite Spitzenwert, der auf dem zweiten Stimulationsstromwert beruht, der durch Verringern des ersten Stimulationsstromwerts um den dem Stromschrittwert entsprechenden Wert auf Grundlage des zweiten Stromwertänderungsprozesses erhalten wird, miteinander verglichen werden (ST776),
wenn bestimmt wird, dass der zweite Spitzenwert gegenüber dem ersten Spitzenwert verringert ist, der Kalibrierungsverarbeitungsabschnitt (ST777) den ersten Stimulationsstromwert als den Stimulationsstromwert der maximalen Stimulation erfasst.

8. Vorrichtung zur Überwachung der Muskelentspannung nach einem der Ansprüche 1 bis 7, wobei
die Vorrichtung eine Konfiguration aufweist, bei der
der Kalibrierungsverarbeitungsabschnitt in einer Elektromyographie angeordnet ist, die einen Muskelentspannungszustand der Person auf Grundlage eines Aktionspotenzials von Muskelfasern eines Muskels überwacht, das induziert wird, wenn eine elektrische Stimulation auf einen Nerv der Person angewendet wird.

9. Vorrichtung zur Überwachung der Muskelentspannung nach Anspruch 1, wobei der Stromwertänderungsprozess zwei Prozessmodi eines Stromwerterhöhungsprozesses und eines Stromwertverringerungsprozesses umfasst.

10. Vorrichtung zur Überwachung der Muskelentspannung nach Anspruch 1, wobei der vorbestimmte Stimulationszeitpunkt 0,5 Sekunden umfasst.

11. Kalibrierungsverarbeitungsverfahren zum Ermitteln eines Stimulationsstromwerts einer supramaximalen Stimulation, die eine maximale Stimulation eines Muskels einer Person überschreitet, wobei das Verfahren umfasst:
einen Prozess des Bestimmens (ST75), als einen Stromwertänderungsprozess, eines der folgenden Prozesse: eines ersten Stromwertänderungsprozesses (ST76), der zu einem vorbestimmten Stimulationszeitpunkt um einen einem vorbestimmten Stromschrittwert entsprechenden Wert erhöht; und eines zweiten Stromwertänderungsprozesses (ST77), der zu dem Stimulationszeitpunkt um den dem Stromschrittwert entsprechenden Wert verringert, wobei die Bestimmung (ST75) auf einem Ergebnis eines Vergleichs eines Amplitudenspitzenwerts des elektrischen Signals, das auf der Stimulationsantwort des Muskels infolge der elektrischen Stimulation mit dem anfänglichen Stimulationsstromwert beruht, und eines Amplitudenspitzenwerts des elektrischen Signals beruht, das auf der Stimulationsantwort des Muskels infolge der elektrischen Stimulation mit dem Stimulationsstromwert beruht, der durch Verringern des anfänglichen Stimulationsstromwerts um den dem Stromschrittwert entsprechenden Wert erhalten wird;
einen Prozess des Einstellens (ST71) eines anfänglichen Stimulationsstromwerts, der so eingestellt ist, dass er kleiner als ein Maximalwert eines Stimulationsstromwerts in einer Vorrichtungskonfiguration gemäß Informationen über die Person ist, als einen Ausgangsstimulationsstromwert, und des Erfassens (ST762, ST772), als einen ersten Spitzenwert, eines Amplitudenspitzenwerts eines elektrischen Signals, das auf einer Stimulationsantwort des Muskels infolge einer elektrischen Stimulation mit einem ersten Stimulationsstromwert beruht, bei dem es sich um einen Stimulationsstromwert handelt, der erhalten wird, bevor der Stimulationsstromwert in dem bestimmten Stromwertänderungsprozess um den dem Stromschrittwert entsprechenden Wert geändert wird;
einen Prozess des Erfassens (ST72), als einen zweiten Spitzenwert, eines Amplitudenspitzenwerts eines elektrischen Signals, das auf einer Stimulationsantwort des Muskels infolge einer elektrischen Stimulation mit einem zweiten Stimulationsstromwert beruht, der auf Grundlage des bestimmten Stromwertänderungsprozesses um den dem Stromschrittwert entsprechenden Wert gegenüber dem ersten Stimulationsstromwert geändert wird, und zwar zu einem Stimulationszeitpunkt, der auf den Stimulationszeitpunkt des ersten Stimulationsstromwerts folgt; und
einen Prozess des Erfassens (ST767, ST777), auf Grundlage eines Ergebnisses eines Vergleichs des ersten Spitzenwerts und des zweiten Spitzenwerts, eines Stimulationsstromwerts einer maximalen Stimulation des Muskels der Person und des Ermittelns (ST768, 778) eines Stimulationsstromwerts, der durch Addieren des Stromschrittwerts zu dem Stimulationsstromwert der maximalen Stimulation der Person erhalten wird, als den Stimulationsstromwert der supramaximalen Stimulation der Person.

12. Kalibrierungsverarbeitungsverfahren nach Anspruch 11, wobei der Stromwertänderungsprozess zwei Prozessmodi eines Stromwerterhöhungsprozesses und eines Stromwertverringerungsprozesses umfasst.

13. Kalibrierungsverarbeitungsverfahren nach Anspruch 11, wobei der vorbestimmte Stimulationszeitpunkt 0,5 Sekunden umfasst.

## Revendications

1. Appareil de surveillance de relâchement musculaire comprenant
une section de traitement d'étalonnage destinée à acquérir une valeur de courant de stimulation d'une stimulation supramaximale dépassant une stimulation maximale d'un muscle d'un sujet,
dans lequel la section de traitement d'étalonnage est configurée pour :
régler (ST71), comme valeur de départ du courant de stimulation, une valeur initiale du courant de stimulation qui est réglée de manière à être inférieure à une valeur maximale d'un courant de stimulation dans une configuration de l'appareil conformément à des informations relatives au sujet ;
déterminer (ST75), en tant que processus de variation de la valeur de courant, l'un des processus suivants : un premier processus de variation de la valeur de courant (ST76) qui augmente d'une valeur correspondant à une valeur prédéterminée de pas de courant à un instant de stimulation prédéterminé ; et un deuxième processus de variation de la valeur de courant (ST77) qui diminue de la valeur correspondant à la valeur de pas de courant à l'instant de stimulation, la détermination (ST75) étant basée sur un résultat d'une comparaison entre une valeur de crête d'amplitude du signal électrique qui est basée sur la réponse à la stimulation du muscle due à la stimulation électrique avec la valeur initiale du courant de stimulation, et une valeur de crête d'amplitude du signal électrique qui est basée sur la réponse à la stimulation du muscle due à la stimulation électrique avec la valeur du courant de stimulation qui est obtenue en diminuant la valeur initiale du courant de stimulation de la valeur correspondant à la valeur de pas de courant ;
détecter (ST72), comme première valeur de crête, une valeur de crête d'amplitude d'un signal électrique qui est basée sur une réponse à la stimulation du muscle due à une stimulation électrique avec une première valeur du courant de stimulation qui est une valeur du courant de stimulation obtenue avant que la valeur du courant de stimulation ne soit modifiée de la valeur correspondant à la valeur de pas de courant dans le processus déterminé de variation de la valeur de courant ;
détecter (ST765, ST775), comme deuxième valeur de crête, une valeur de crête d'amplitude d'un signal électrique qui est basée sur une réponse à la stimulation du muscle due à une stimulation électrique avec une deuxième valeur du courant de stimulation qui est modifiée de la valeur correspondant à la valeur de pas de courant par rapport à la première valeur du courant de stimulation, sur la base du processus déterminé de variation de la valeur de courant, à un instant de stimulation qui suit l'instant de stimulation de la première valeur du courant de stimulation ; et
détecter (ST767, ST777), sur la base d'un résultat d'une comparaison entre la première valeur de crête et la deuxième valeur de crête, une valeur du courant de stimulation d'une stimulation maximale du muscle du sujet, et acquérir (ST768, ST778) une valeur du courant de stimulation qui est obtenue en ajoutant la valeur de pas de courant à la valeur du courant de stimulation de la stimulation maximale du sujet, comme valeur du courant de stimulation de la stimulation supramaximale du sujet.

2. Appareil de surveillance de relâchement musculaire selon la revendication 1, dans lequel la valeur initiale du courant de stimulation est une valeur allant de 10 mA à une valeur inférieure à 60 mA.

3. Appareil de surveillance de relâchement musculaire selon la revendication 1 ou 2, dans lequel la valeur initiale du courant de stimulation est réglée sur la base d'informations relatives au sujet.

4. Appareil de surveillance de relâchement musculaire selon l'une quelconque des revendications 1 à 3, dans lequel la valeur de pas de courant est réglée sur la base de la valeur initiale du courant de stimulation.

5. Appareil de surveillance de relâchement musculaire selon l'une quelconque des revendications 1 à 4, dans lequel l'instant de stimulation est réglé dans une plage de 2 à 4 Hz.

6. Appareil de surveillance de relâchement musculaire selon l'une quelconque des revendications 1, 4 et 5, dans lequel,
dans le cas où est exécuté un processus d'augmentation/comparaison de la valeur de courant dans lequel la première valeur de crête qui est basée sur la première valeur du courant de stimulation, qui est une valeur du courant de stimulation obtenue avant l'augmentation de la valeur du courant de stimulation, et la deuxième valeur de crête qui est basée sur la deuxième valeur du courant de stimulation, qui est obtenue en augmentant la première valeur du courant de stimulation de la valeur correspondant à la valeur de pas de courant sur la base du premier processus de variation de la valeur de courant, sont comparées (ST766) l'une à l'autre,
lorsqu'il est déterminé que la première valeur de crête et la deuxième valeur de crête sont équivalentes l'une à l'autre, la section de traitement d'étalonnage détecte (ST767) la première valeur du courant de stimulation comme valeur du courant de stimulation de la stimulation maximale.

7. Appareil de surveillance de relâchement musculaire selon l'une quelconque des revendications 1 et 4 à 6, dans lequel,
dans le cas où est exécuté un processus de diminution/comparaison de la valeur de courant dans lequel la première valeur de crête qui est basée sur la première valeur du courant de stimulation, qui est une valeur du courant de stimulation obtenue avant la diminution de la valeur du courant de stimulation, et la deuxième valeur de crête qui est basée sur la deuxième valeur du courant de stimulation, qui est obtenue en diminuant la première valeur du courant de stimulation de la valeur correspondant à la valeur de pas de courant sur la base du deuxième processus de variation de la valeur de courant, sont comparées (ST776) l'une à l'autre,
lorsqu'il est déterminé que la deuxième valeur de crête est diminuée par rapport à la première valeur de crête, la section de traitement d'étalonnage détecte (ST777) la première valeur du courant de stimulation comme valeur du courant de stimulation de la stimulation maximale.

8. Appareil de surveillance de relâchement musculaire selon l'une quelconque des revendications 1 à 7, dans lequel
l'appareil présente une configuration dans laquelle
la section de traitement d'étalonnage est disposée dans une électromyographie qui surveille un état de relâchement musculaire du sujet sur la base d'un potentiel d'action de fibres musculaires d'un muscle qui est induit lorsqu'une stimulation électrique est appliquée à un nerf du sujet.

9. Appareil de surveillance de relâchement musculaire selon la revendication 1, dans lequel le processus de variation de la valeur de courant comprend deux modes de processus, à savoir un processus d'augmentation de la valeur de courant et un processus de diminution de la valeur de courant.

10. Appareil de surveillance de relâchement musculaire selon la revendication 1, dans lequel l'instant de stimulation prédéterminé comprend 0,5 seconde.

11. Procédé de traitement d'étalonnage destiné à acquérir une valeur du courant de stimulation d'une stimulation supramaximale dépassant une stimulation maximale d'un muscle d'un sujet, dans lequel le procédé comprend :
un processus de détermination (ST75), en tant que processus de variation de la valeur de courant, de l'un des processus suivants : un premier processus de variation de la valeur de courant (ST76) qui augmente d'une valeur correspondant à une valeur prédéterminée de pas de courant à un instant de stimulation prédéterminé ; et un deuxième processus de variation de la valeur de courant (ST77) qui diminue de la valeur correspondant à la valeur de pas de courant à l'instant de stimulation, la détermination (ST75) étant basée sur un résultat d'une comparaison entre une valeur de crête d'amplitude du signal électrique qui est basée sur la réponse à la stimulation du muscle due à la stimulation électrique avec la valeur initiale du courant de stimulation, et une valeur de crête d'amplitude du signal électrique qui est basée sur la réponse à la stimulation du muscle due à la stimulation électrique avec la valeur du courant de stimulation qui est obtenue en diminuant la valeur initiale du courant de stimulation de la valeur correspondant à la valeur de pas de courant ;
un processus de réglage (ST71), comme valeur de départ du courant de stimulation, d'une valeur initiale du courant de stimulation qui est réglée de manière à être inférieure à une valeur maximale d'une valeur du courant de stimulation dans une configuration d'appareil conformément à des informations relatives au sujet, et de détection (ST762, ST772), comme première valeur de crête, d'une valeur de crête d'amplitude d'un signal électrique qui est basée sur une réponse à la stimulation du muscle due à une stimulation électrique avec une première valeur du courant de stimulation qui est une valeur du courant de stimulation obtenue avant que la valeur du courant de stimulation ne soit modifiée de la valeur correspondant à la valeur de pas de courant dans le processus déterminé de variation de la valeur de courant ;
un processus de détection (ST72), comme deuxième valeur de crête, d'une valeur de crête d'amplitude d'un signal électrique qui est basée sur une réponse à la stimulation du muscle due à une stimulation électrique avec une deuxième valeur du courant de stimulation qui est modifiée de la valeur correspondant à la valeur de pas de courant par rapport à la première valeur du courant de stimulation, sur la base du processus déterminé de variation de la valeur de courant, à un instant de stimulation qui suit l'instant de stimulation de la première valeur du courant de stimulation ; et
un processus de détection (ST767, ST777), sur la base d'un résultat d'une comparaison entre la première valeur de crête et la deuxième valeur de crête, d'une valeur du courant de stimulation d'une stimulation maximale du muscle du sujet, et d'acquisition (ST768, 778) d'une valeur du courant de stimulation qui est obtenue en ajoutant la valeur de pas de courant à la valeur du courant de stimulation de la stimulation maximale du sujet, comme valeur du courant de stimulation de la stimulation supramaximale du sujet.

12. Procédé de traitement d'étalonnage selon la revendication 11, dans lequel le processus de variation de la valeur de courant comprend deux modes de processus, à savoir un processus d'augmentation de la valeur de courant et un processus de diminution de la valeur de courant.

13. Procédé de traitement d'étalonnage selon la revendication 11, dans lequel l'instant de stimulation prédéterminé comprend 0,5 seconde.
